(19) Europäisches Patentamt / European Patent Office / Office européen des brevets

(11) **EP 4 276 461 B1**

(12) **FASCICULE DE BREVET EUROPEEN**

(45) Date de publication et mention
de la délivrance du brevet:
**17.07.2024   Bulletin 2024/29**

(21) Numéro de dépôt: **23171737.2**

(22) Date de dépôt: **04.05.2023**

(51) Classification Internationale des Brevets (IPC):
***G01N 33/24*** *(2006.01)*

(52) Classification Coopérative des Brevets (CPC):
**G01N 33/241**

(54) **PROCÉDÉ POUR L'ANALYSE DE LA COMPOSITION D'HYDROCARBURES AU MOYEN D'UN PYROLYSEUR SANS DISPOSITIF DE SÉPARATION**

VERFAHREN ZUR ANALYSE DER KOHLENWASSERSTOFFZUSAMMENSETZUNG MITTELS EINES PYROLYSATORS OHNE TRENNVORRICHTUNG

METHOD FOR ANALYZING HYDROCARBON COMPOSITION USING PYROLYZER WITHOUT SEPARATION DEVICE

(84) Etats contractants désignés:
**AL AT BE BG CH CY CZ DE DK EE ES FI FR GB GR HR HU IE IS IT LI LT LU LV MC ME MK MT NL NO PL PT RO RS SE SI SK SM TR**

(30) Priorité:  **09.05.2022   FR 2204374**

(43) Date de publication de la demande:
**15.11.2023   Bulletin 2023/46**

(73) Titulaire: **Vinci Technologies**
**92000 Nanterre (FR)**

(72) Inventeurs:
• **Ammouial, Jérémie**
**92100 Boulogne Billancourt (FR)**
• **Bouton, Nicolas**
**94800 Villejuif (FR)**

(74) Mandataire: **Alatis**
**3, rue Paul Escudier**
**75009 Paris (FR)**

(56) Documents cités:
**EP-B1- 0 691 540      WO-A1-2005/111603**
**WO-A1-2020/157389**

**Description**

La présente invention concerne un procédé de caractérisation en continu d'une composition d'huiles et de pétroles bruts pouvant être analysés à l'état libre ou en étant contenus dans une roche sédimentaire.

Les paramètres recherchés par le procédé de l'invention comprennent : la composition en termes d'hydrocarbures, le poids moléculaire moyen de l'échantillon et le point d'ébullition (dit « True boiling point »).

Les applications visées par l'invention sont situées principalement dans les domaines de l'industrie pétrolière, notamment, en phase d'exploration et en phase de raffinage. Plus précisément, l'invention s'intéresse à la détermination, lors des premières études de prospection à l'échelle du bassin sédimentaire, de la composition des hydrocarbures contenus dans les roches mères ou dans les roches réservoirs, à partir de débris de roches (ou « cuttings ») arrachés par l'outil de forage et remontés à la surface par le fluide de forage.

En effet, la nature géologique de ces débris, leurs caractéristiques physiques et leur teneur en divers fluides (notamment, eau, hydrocarbures et autres gaz, ...) peuvent fournir des informations précieuses sur les roches forées sans qu'il soit nécessaire d'extraire ces fluides des échantillons prélevés, en un temps réduit et de façon automatique. Toutefois, l'invention peut aussi concerner d'autres domaines d'applications comme l'analyse des sites et sols qui ont été pollués par des hydrocarbures.

Le pétrole a pour origine le kérogène contenu dans les roches mères et issu de la matière organique présente dans le milieu au moment de la sédimentation de ladite roche mère (RM). Selon le milieu de dépôt de cette roche mère, le kérogène résultant pourra être de trois types différents. Le type I correspondant à un milieu lacustre dans lequel la matière organique produite est enrichie en lipide. Le type II correspond à un milieu de dépôt de type marin dans lequel la matière organique précurseur est composée à la fois de lipides et de cellulose. La matière organique de type III quant à elle correspond à un environnement de dépôt de type continental avec une matière organique d'origine enrichie en cellulose et lignine. En fonction du type de kérogène la composition du pétrole créé par craquage thermique du kérogène, lors de l'enfouissement de la roche mère au cours des temps géologiques, va être amené à varier. La qualité du pétrole en est alors directement affectée.

Le taux d'avancement du craquage thermique du kérogène considéré est également un paramètre fondamental à prendre en considération lors de l'étude de la composition d'un pétrole. En effet, aux prémices du craquage thermique du kérogène, le pétrole obtenu est lourd (c'est-à-dire composés de nombreux hydrocarbures à longues chaînes de carbones) ce qui correspond à l'entrée de la roche mère dans la fenêtre à huiles (fenêtre de températures correspondant à une génération d'hydrocarbures liquides issus du kérogène). Puis, lors de l'augmentation de la profondeur de la roche mère due au phénomène de subsidence, le craquage thermique produit des hydrocarbures à chaînes de plus en plus courtes issues du kérogène jusqu'à atteindre la fenêtre à gaz sec dans laquelle l'unique hydrocarbure produit est le plus petit qui soit c'est-à-dire le méthane. Dans un second temps les hydrocarbures lourds, qu'ils aient migré ou non de la roche mère, peuvent subir un second craquage thermique appelé craquage secondaire si la température environnante est suffisamment élevée.

Le dernier facteur important permettant d'expliquer la qualité du pétrole considéré est sa préservation au cours des temps géologiques. En effet, si les hydrocarbures sont ramenés en proche surface, la qualité peut en être diminuée du fait d'organismes de type archée pouvant vivre jusqu'à des températures supérieures à 100°C et se nourrissant de n-alcanes. La conséquence directe est une diminution drastique du degré API du pétrole (augmentation du sa densité) ainsi qu'un enrichissement en molécules possédant des hétéroatomes comprenant de l'azote, du soufre et de l'oxygène (NSO).

L'ensemble de ces paramètres permet d'expliquer la grande variabilité de la composition des pétroles présents sur la terre. Ainsi, l'invention fournira aux utilisateurs des données essentielles dès le commencement des forages exploratoires sur la composition du pétrole présent sur le site prospecté.

On connaît la technique dite Rock-Eval (marque déposée - IFP Energies nouvelles) décrite notamment dans les brevets FR2472754 et EP0691540B1. Cette technique d'analyse, qui est rapide et quasiment automatique, a été développée pour la caractérisation de la matière organique et des hydrocarbures contenus dans un échantillon de roche provenant d'une formation géologique. Plus précisément, cette technique permet de déterminer la présence, la nature et le degré de maturité de la matière organique contenue dans un échantillon de roche.

La méthode Rock-Eval fournit également des informations précises sur la quantification des hydrocarbures, la quantité de carbone organique total (TOC) et la quantité de carbone minéral (MinC) contenus dans un échantillon de roche. Ainsi, la technique Rock-Eval permet en particulier de mesurer la quantité de composés hydrocarbonés libérés tout au long de la pyrolyse. On peut alors établir un pyrogramme qui est une courbe représentant l'évolution de la quantité de composés hydrocarbonés libérés rapportée au poids de l'échantillon considéré en fonction du temps. Un pyrogramme présente généralement plusieurs pics. Les pics sont bien différenciés et la surface de chaque pic est calculée. On obtient ainsi, pour chaque pic, une grandeur représentative de la quantité de composés hydrocarbonés libérés sur une plage de températures encadrant le pic considéré.

Deux méthodes principales mettant en oeuvre deux séquences de températures différentes ont été développées. La méthode « Basique » (dite « Basic method » ou « Bulk Rock method » en anglais), dédiée plus particulièrement aux échantillons de roches-mères, est décrite par exemple dans Lafargue et al (1998) et Behar et al (2001). La méthode « Réservoir » (dite « Réservoir method » en anglais), dédiée plus particulièrement aux échantillons de roches réservoir, est par exemple décrite dans le brevet EP0691540B1.

Le brevet EP3918321 décrit une méthode d'analyse des hydrocarbures gazeux en C1-C6 au moyen d'un analyseur multi-gaz permettant la quantification desdits hydrocarbures gazeux. Cependant cette méthode ne permet pas la quantification des hydrocarbures comportant plus de 6 carbones car le transport de ces hydrocarbures vers l'analyseur infra-rouge se fait uniquement à l'état gazeux aux conditions de pression et de températures atmosphériques, ce qui ne correspond pas à l'état physique des hydrocarbures comportant plus de six carbones.

En outre, il s'est avéré nécessaire d'améliorer la précision des résultats obtenus avec le procédé Rock-Eval® et d'en optimiser l'interprétation afin que les décisions économiques et techniques relatives à l'exploration pétrolière reposent sur des bases plus solides. En effet, depuis les débuts de la mise en oeuvre du procédé Rock-Eval, de nombreuses méthodes de caractérisation des hydrocarbures thermo-vaporisables contenus dans les roches ont été développées mais aucune n'a permis de décrire précisément la composition de ces hydrocarbures. Les méthodes existantes (décrites précédemment ainsi que d'autres développées plus récemment) permettent tout au plus d'estimer les poids moléculaires relatifs de chaque fraction d'hydrocarbures analysés en tentant de segmenter le signal des détecteurs à ionisation de flamme (FID) via des paliers de températures déterminés arbitrairement et sans réelle consistance physique.

C'est dans ce contexte que l'invention a cherché à développer un nouveau procédé permettant de résoudre les problèmes techniques posés par les méthodes antérieures afin de quantifier ces hydrocarbures liquides et solides aux conditions atmosphériques au moyen d'un procédé simple et rapide permettant en peu d'étapes et à partir d'un échantillon de faible quantité et de diverses natures, d'obtenir des résultats fiables et inédits.

Le principe physique à la base du procédé de caractérisation de l'invention repose sur la loi d'Antoine (simplification de la loi thermodynamique de Clausius-Clapeyron) qui décrit l'évolution de la pression de vapeur saturante avec l'élévation de température lorsque l'équilibre liquide-vapeur est atteint. C'est cette loi physique qui intervient principalement lors de l'élévation de la température au cours de la pyrolyse d'un échantillon de roches contenant des hydrocarbures libres.

En effet, l'analyse d'une quantité très faible d'hydrocarbures (quelques microlitres au maximum) contenue dans les roches nécessite l'utilisation de la loi d'Antoine pour relier la masse d'hydrocarbure évaporé (c'est-à-dire étant passée de la phase liquide à la phase gazeuse) à la température de ce changement de phase.

Par exemple, si l'on analyse du n-décane pur (dont la température d'ébullition est de 174.1°C) en pyrolyse via une élévation de température à taux constant, la température du maximum d'évaporation du n-décane sera inférieure à la température d'ébullition théorique (entre 70 et 130°C selon la quantité de produite analysé). Ce constat est également accentué par un second phénomène occurrent lors de l'évaporation d'un composé hydrocarboné, à savoir la diffusion de la matière, qui est régie par la loi de Fick, à travers une interface dont le flux est dirigé en direction inverse au gradient de concentration (dans le sens des valeurs de concentrations décroissantes).

Pour chaque n-paraffine (hydrocarbures entre C1 et C120), les courbes d'évaporation reliant la masse des hydrocarbures distillés à la température du four de pyrolyse ont été modélisées à l'aide de la loi thermodynamique d'Antoine. Cette approche a alors permis de créer un abaque donnant la position de chaque hydrocarbure selon son poids moléculaire.

Ainsi, l'objet de l'invention est un procédé pour la caractérisation et la quantification en continu des hydrocarbures en C1-C120 comprenant la pyrolyse sous atmosphère non oxydante de l'échantillon, l'entraînement des effluents gazeux de pyrolyse au moyen d'un courant de gaz inerte puis l'analyse desdits effluents en vue d'obtenir leurs profils en fonction de la température de pyrolyse, caractérisé en ce qu'on superpose lesdits profils sur l'abaque de référence créé à partir de la loi physique de Clausius Clapeyron pour déterminer la composition des hydrocarbures dans l'échantillon analysé.

Plus précisément, le procédé expérimental de l'invention consiste en une élévation de température sous atmosphère inerte à vitesse constante dudit échantillon, d'une température T1 la plus faible possible (c'est-à-dire à température ambiante voir plus bas si les moyens techniques le permettent) à une température finale T2 nécessairement supérieure à la température d'ébullition la plus élevée du mélange à analyser par ledit procédé. La température T1 est donc nécessairement inférieure à la température T2.

Selon une caractéristique avantageuse du procédé de l'invention, on élève la température de la pyrolyse avec un unique gradient de température compris entre 0,1 et 50°C/min. De préférence, ce gradient de température est compris entre 10 et 30 °C/min.

Selon une autre caractéristique du procédé de l'invention, on effectue la pyrolyse avec une vitesse de chauffe constante comprise entre 0,5°C/mn et 50°C/mn pour atteindre une température finale comprise entre 30°C et 900°C.

De préférence et selon une variante de mise en oeuvre du procédé de l'invention, on effectue la pyrolyse selon une vitesse de chauffe comprise entre 15°C/mn et 25°C/min pour atteindre une température finale comprise entre 640°C et 660°C.

Par ailleurs, on effectue la dé-convolution des profils mesurés par l'équation d'Antoine de façon à obtenir les proportions des classes d'isomères et les courbes de distillation simulée de l'échantillon analysé ainsi que le diagramme de répartition desdites classes d'isomères.

De préférence, les effluents gazeux de pyrolyse sont entraînés et dirigés par le courant de gaz inerte avec un débit compris entre 20ml/mn et 70ml/mn.

La mise en oeuvre dudit procédé permet d'obtenir la composition du mélange d'hydrocarbures contenus dans ledit échantillon de roche ainsi que la masse molaire moyenne et le degré API via le traitement des données par l'abaque dont chacune des courbes le composant (allant de n-C1 à n-C120) est calculé théoriquement par la formule (F) suivante.

$$m_i = (D_v \times M_i \times t)/(R \times T) \times 10^{A-B/(T+C)}$$

Avec

$x_i$ (-) : la fraction molaire de l'hydrocarbure considéré
$m_i$ (kg) : la masse de l'hydrocarbure considéré
$D_v$ (m$^3$.s$^{-1}$): le débit volumique du gaz vecteur
$M_i$ (kg.mol$^{-1}$) : la masse molaire de l'hydrocarbure considéré
$T$ (K): la température
$t$ (s) : le temps
$R$ (J.mol$^{-1}$.K$^{-1}$): la constante des gaz parfaits
$A$ (Pa), $B$ (-) et $C$ (K) : les paramètres de l'équation d'Antoine
$P_i$ (Pa): la pression partielle
$P_{tot}$ (Pa) : la pression totale du système.

L'axe des abscisses dudit abaque à pour grandeur la température tandis que l'axe des ordonnées à pour unité la masse. Ainsi, de façon synthétique, le procédé selon l'invention pour l'analyse du pétrole libre ou contenu dans un échantillon de roche comprend une phase de pyrolyse de l'échantillon effectuée sous atmosphère inerte dans une plage de températures croissantes comprises entre une température initiale T1 et une température finale T2 selon une augmentation linéaire de la température avec une cinétique comprise entre 0,1°C/min et 50°C/min. Les hydrocarbures vaporisés issus de la pyrolyse de l'échantillon sont quantifiés et caractérisés grâce à leurs températures de vaporisation. L'évolution de ces dernières étant prédites théoriquement par la loi d'Antoine, le signal d'hydrocarbure obtenu peut dès lors être segmenté en différentes classe d'isomères de C1 à C120.

Ledit procédé peut-être optionnellement suivi d'une phase oxydante afin de déterminer le COT (pourcentage de carbone organique total) dans le cas de l'analyse d'huiles lourdes.

Le procédé de l'invention peut intégrer des logiciels performants qui effectuent le tracé des courbes correspondant aux profils des hydrocarbures, les calculs quantitatifs, la détermination des distributions de ces hydrocarbures et qui permettent d'obtenir des informations sur les quantités d'hydrocarbures obtenues par pyrolyse, ainsi que des données inédites sur leur genèse.

La mise en oeuvre du procédé de l'invention vient compléter et prolonger la détection des hydrocarbures gazeux en C1-C6 qui peut être effectuée via, par exemple, la méthode décrite dans le brevet EP3918321. En effet, la température minimale de début d'analyse de 30°C prévue dans le procédé de l'invention n'est pas adaptée à l'obtention de résultats d'analyse des hydrocarbures en dessous de C7.

D'autres caractéristiques et avantages du procédé selon l'invention, apparaîtront à la lecture de la description ci-après d'exemples non limitatifs de mise en oeuvre, en se référant aux figures annexées et décrites ci-après.

[FIG. 1] est une vue schématique d'un mode de réalisation préférentiel du dispositif destiné à la mise en oeuvre du procédé de l'invention.

[FIG. 2] illustre la séquence de variation de la température selon le procédé de l'invention.

[FIG. 3] est un graphique/abaque créé à partir de l'équation d'Antoine appliqué au four de pyrolyse du Rock-Eval, selon une mise en oeuvre particulière du procédé de l'invention, permettant de prédire la quantité d'alcane pour les isomères de C1 à C40.

[FIG. 4] représente une superposition de l'abaque et d'une courbe d'hydrocarbures obtenue via la séquence de température sur un échantillon pétrolier selon un mode de mise en oeuvre du procédé de l'invention.

[FIG. 5] est une superposition des courbes expérimentales obtenues via l'analyse par Rock-Eval de n-C10, n-C20, n-C30 et n-C40 à différentes masses avec des courbes de distillation simulées théoriques de ces mêmes paraffines obtenues via l'équation d'Antoine.

[FIG. 6] représente une répartition des isomères d'un pétrole brut et de deux huiles de référence en pourcentages. Les barres noires sont les proportions obtenues à partir de la distillation simulation Rock-Eval tandis que les barres hachurées ont été obtenues via une analyse par chromatographie en phase gazeuse.

[FIG. 7] représente une autre répartition des isomères d'un pétrole brut et de deux huiles de référence en pourcentages. Les barres noires sont les proportions obtenues à partir de la distillation simulation Rock-Eval tandis que les barres hachurées ont été obtenues via une analyse par chromatographie en phase gazeuse.

[FIG. 8] représente encore une autre répartition des isomères d'un pétrole brut et de deux huiles de référence en pourcentages. Les barres noires sont les proportions obtenues à partir de la distillation simulation Rock-Eval tandis que les barres hachurées ont été obtenues via une analyse par chromatographie en phase gazeuse.

[FIG. 9] représente les courbes de distillation simulée par Rock-Eval (courbes trait plein noires) pour un pétrole brut et pour deux huiles de référence ainsi que les courbes de distillation simulée par chromatographie gazeuse (courbes pointillées).

L'invention concerne, de manière générale, un procédé destiné à la caractérisation des hydrocarbures contenus dans un échantillon de roche. Ce procédé est caractérisé par une élévation linéaire de la température sous atmosphère inerte selon un coefficient directeur choisi par l'utilisateur et compris entre 0,1°C et 50°C par minute.

En particulier, l'invention concerne l'analyse des fractions d'hydrocarbures thermo-vaporisables et, s'intéresse, notamment, à leur identification par la température. En effet, il est bien connu que les hydrocarbures les plus légers sont vaporisés avant les plus lourds. Toutefois, les quantités d'hydrocarbures analysés étant très faibles avec la méthode du type Rock-Eval, les températures auxquelles sont détectés ces hydrocarbures ne correspondent pas aux températures d'ébullition théoriques des composants de l'échantillon. Depuis l'apparition de la méthode Rock-Eval, qui a permis de quantifier en un temps remarquable les hydrocarbures contenus dans les roches mères pétrolières, la détermination de la composition des hydrocarbures thermo-vaporisables contenus dans ces roches est devenue essentielle car cette composition constitue un critère de qualité primordiale du pétrole que ces roches contiennent. En effet, les estimations de l'investissement financier à fournir pour mettre en production le gisement pétrolier prospectif ne seront pas les mêmes si les hydrocarbures présents sont légers ou lourds.

Le principal problème posé par la détermination de la composition de mélanges d'hydrocarbures avec la méthode Rock-Eval et les pyrolyseurs apparentés résidait en l'absence de dispositif de séparation comme, par exemple, lors de l'analyse chromatographique. De plus, bien que les températures lues par le dispositif Rock-Eval soient précises, elles ne correspondent pas aux températures d'ébullition réelles des composés analysés car ces derniers se trouvent présents en trop faibles quantités.

L'invention et son procédé de mise en oeuvre décrit ci-après permettent de palier à cette problématique grâce à l'application de la loi de Clausius-Clapeyron et, en particulier, de la loi d'Antoine décrivant le passage des hydrocarbures de la phase liquide à la phase vapeur au cours d'une élévation de température. Ce procédé peut être avantageusement, mais non limitativement, mis en oeuvre au moyen du dispositif Rock-Eval décrit dans le brevet EP2342557.

Le procédé de l'invention est appliqué principalement à des échantillons de roches pétrolières de type roches réservoirs, roches mères et pétroles bruts, mais peut être également utilisé pour des sols pollués aux hydrocarbures ou bien encore pour les catalyseurs issus des procédés de raffinage. Plus généralement l'invention peut concerner tout échantillon contenant des hydrocarbures dont il est nécessaire de connaître la composition. Jusqu'à présent, la connaissance de la composition de ces hydrocarbures contenus dans un échantillon nécessitait une extraction via différents solvants puis une analyse chromatographique. L'invention est une avancée en ce qu'elle permet une caractérisation de la composition d'hydrocarbures automatique, rapide et in situ.

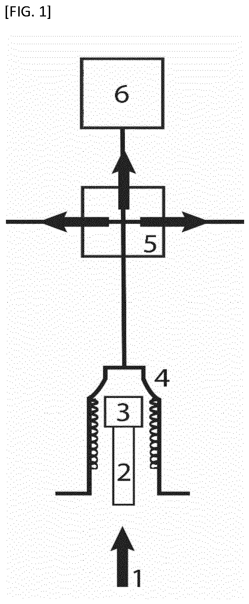

La figure 1 représente, de façon schématique, un mode de réalisation du dispositif pour la mise en oeuvre du procédé. Ce dispositif comprend un four 4 destiné à la pyrolyse dont la température peut aller de 0°C à 900°C. Un échantillon de roche contenu dans une nacelle 3 est alors introduit dans le four par l'intermédiaire d'un piston 2. Le gaz vecteur inerte 1, circulant en permanence à débit constant dans le système, entraîne le produit de la pyrolyse vers le détecteur à ionisation de flamme 6 (FID pour « Flame Ionisation Detector ») permettant alors de quantifier les hydrocarbures. Selon une variante de réalisation, le dispositif peut être muni d'un diviseur chauffant 5 permettant de rediriger le flux de gaz vers d'autres détecteurs.

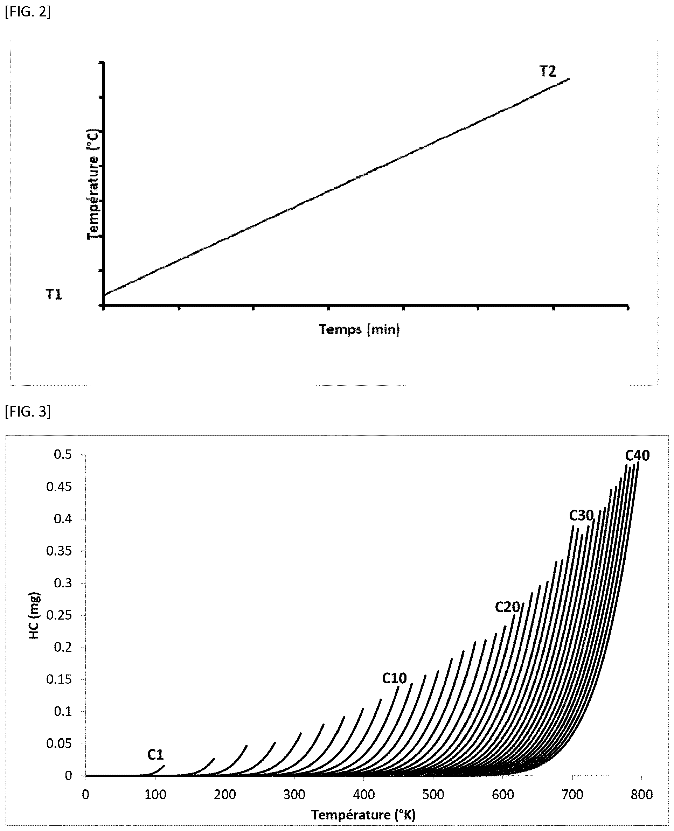

La figure 2 représente l'élévation de la température T1 à la température T2 avec un unique gradient de température compris entre 0,1 et 50°C/min. Par conséquent, cette élévation de température ne comprend aucun palier.

Le flux d'hydrocarbures pyrolysés est entraîné et dirigé par le courant de gaz inerte, dont le débit peut être compris entre 20ml/mn et 70ml/mn, vers un détecteur 6 du type à ionisation de flamme (FID) (figure 1).

Le phénomène de thermo-vaporisation se produisant lors de la pyrolyse de matière organique avec le craquage thermique se décompose en deux phases. La première phase est prédominante de 0°C à 400°C et concerne principalement les hydrocarbures relativement légers (inférieurs à C40) tandis que la seconde phase ne devient prédominante qu'au-delà de 400°C et concerne, en particulier, les hydrocarbures lourds, résines, asphaltènes ainsi que le kérogène.

Une fois l'étape de pyrolyse achevée, on obtient la courbe de thermo-vaporisation des hydrocarbures. Cette courbe peut comprendre un ou plusieurs pics selon l'homogénéité de la répartition des températures d'ébullition de ses composés.

Selon un mode d'illustration avantageux, cette courbe pourra être représentée par un graphique de type $m = f(T)$ où $m$ est la masse en mg et $T$ la température en (°K).

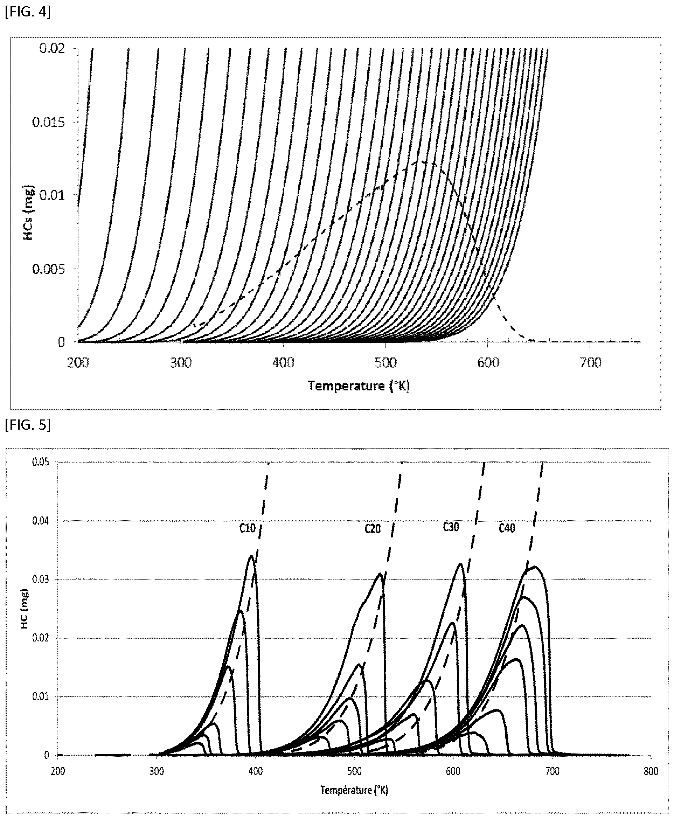

La courbe d'hydrocarbure pourra alors être superposée à l'abaque théorique de vaporisation des n-hydrocarbures aliphatiques comme sur la figure 4.

Les courbes composants l'abaque ont été déterminées grâce à l'application des équations d'Antoine à la pyrolyse selon la méthode Rock-Eval. Lors de l'analyse par la méthode Rock-Eval, le signal obtenu via le détecteur FID (Détecteur à ionisation de flamme) permet de connaître la quantité d'hydrocarbures générée lors de la pyrolyse en fonction de la température. Or, la loi d'Antoine permet d'avoir une relation entre la pression partielle ($P_i$) d'un gaz en fonction de la température (T) dans le cadre d'un équilibre liquide-vapeur.

$$\text{Log}(P_i) = A - (B/(T+C)) \text{ où } P_i = P_{sat}/P_{tot}.$$

Toujours lors de l'analyse par Rock-Eval, l'équilibre n'est jamais atteint car la phase gazeuse est constamment renouvelée, ce qui constitue la principale cause d'erreur ou d'approximation de cette méthode. Afin de faire intervenir la masse d'hydrocarbures qui est mesurée par la méthode Rock-Eval, la pression partielle $P_i$ est ensuite exprimée selon la loi de Raoult.

$$P_i = x_i \times P_{tot}$$

Après avoir fait intervenir la loi des gaz parfaits, la relation (F) suivante est obtenue.

$$m_i = (D_v \times M_i \times t)/(R \times T) \times 10^{A-B/(T+C)}$$

avec $x_i$(-) : la fraction molaire de l'hydrocarbure considéré

$m_i$ (kg) : la masse de l'hydrocarbure considéré

$D_v$ ($m^3.s^{-1}$): le débit volumique du gaz vecteur

$M_i$ ($kg.mol^{-1}$) : la masse molaire de l'hydrocarbure considéré

T (K): la température

t (s) : le temps

R ($J.mol^{-1}.K^{-1}$): la constante des gaz parfaits

A (Pa), B (-) et C (K) : les paramètres de l'équation d'Antoine

$P_i$ (Pa): la pression partielle

$P_{tot}$ (Pa): la pression totale du système.

Ainsi pour chaque n-Paraffine de C1 à C120, il est possible de déterminer les courbes représentées sur la figure 3, de la masse d'hydrocarbures liquides passant sous forme gazeuse en fonction de la température. L'extrémité de chaque courbe correspond à la température d'ébullition théorique de chaque composé. La position relative de chaque courbe dépend de la masse molaire du n-alcane et des paramètres (A-B-C) de l'équation d'Antoine qui lui sont propres.

L'allure générale des courbes est, quant à elle, déterminée par la rampe de variation des températures et le débit volumique utilisé dans le dispositif. Ainsi chaque courbe de l'abaque correspond à l'évaporation de la masse des n-alcanes en fonction de l'augmentation de la température dans le four de pyrolyse. Par superposition de la courbe d'hydrocarbures avec cet abaque, les approximations suivantes sont donc assumées.

- La loi d'Antoine est utilisée alors que l'équilibre entre la phase gazeuse et le liquide n'est pas atteint.
- Chaque isomère se comporte thermiquement comme le n-alcane correspondant.
- Chaque n-alcane se comporte de façon indépendante des autres n-alcanes au sein d'un mélange.

La courbe d'hydrocarbures issue du signal FID est alors segmentée selon un algorithme permettant l'estimation en proportion de chaque classe d'isomères au sein du mélange. L'algorithme employé peut varier selon l'utilisation et les cas d'étude rencontrés.

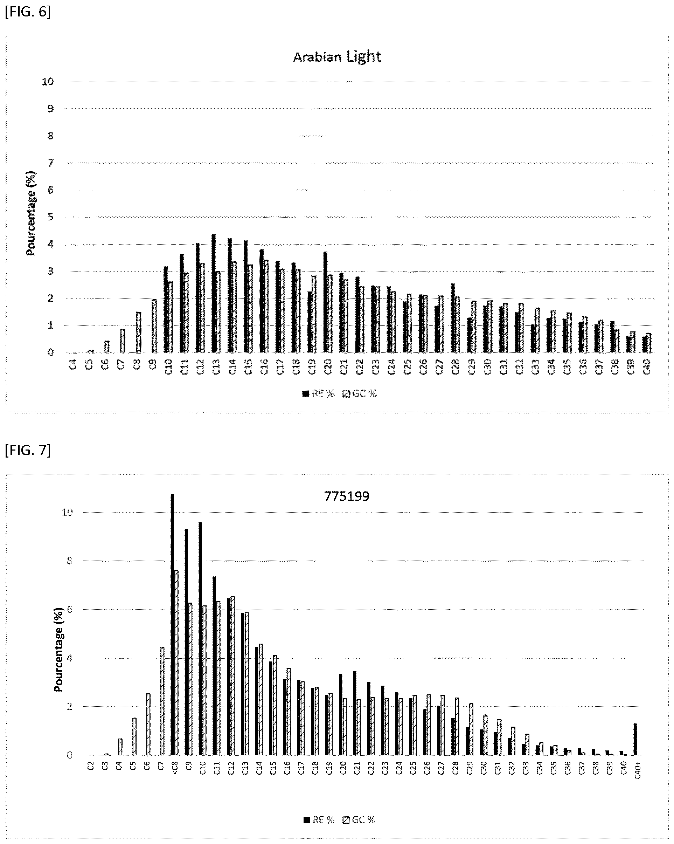

Les proportions de chaque classe d'isomère obtenues par la méthode Rock-Eval RE pour des huiles ont été corrélées avec les proportions des isomères obtenus via la chromatographie en phase gazeuse GC (exemples figures 6 à 8). On peut alors observer que les écarts entre les résultats des deux méthodes sont très faibles. Bien que des biais liés à la méthode de caractérisation du Rock-Eval subsistent, ces derniers sont connus et maîtrisés. En effet, ils sont intimement liés aux approximations énoncées lors de la démonstration des équations de courbes.

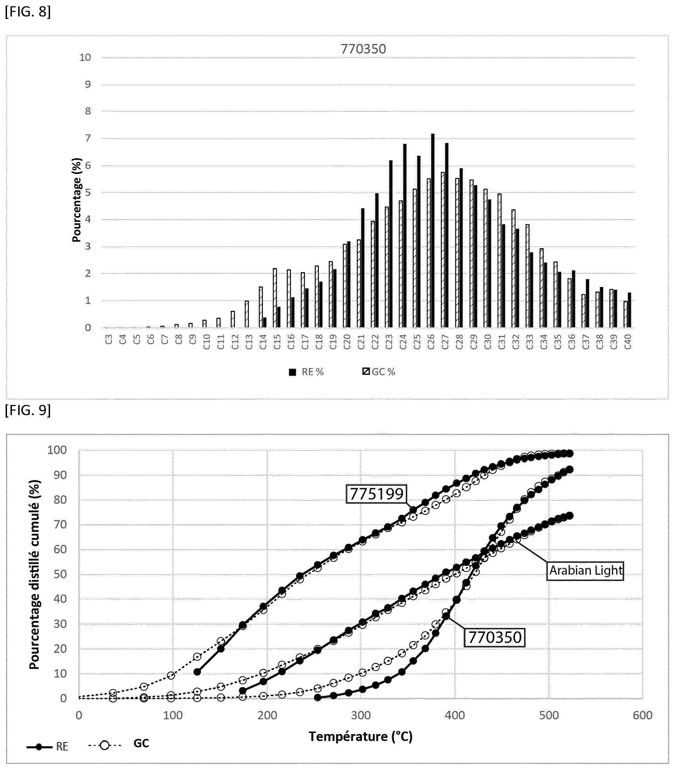

Les proportions de chaque classe d'isomère permettent alors de retracer des courbes de distillation simulées. Cette fois-ci les températures utilisés pour reconstituer ces courbes sont les températures d'ébullition théoriques du n-alcane de la classe d'isomère considérée. Ces courbes de distillation simulée par Rock-Eval (en trait plein) sont alors comparées aux courbes de distillation simulée réalisées par la chromatographie gazeuse (en pointillé) (Figure 9).

L'invention est destinée avant tout à l'analyse des huiles libres et contenues dans les roches réservoirs (poreuses et perméables). L'invention peut-être également utilisée pour l'analyse de roches mères mais peut s'avérer, dans ce cas, moins précise en raison des effets de matrices inhérents à la lithologie argileuse de ces dernières.

Dans la partie suivante, trois échantillons d'huiles (un de pétrole brut et deux d'huiles de références dont les températures d'ébullitions sont connues) ont été analysés via la méthode de distillation simulée Rock-Eval puis en chromatographie gazeuse afin d'en comparer les résultats.

La première huile (de référence 775199) est une huile dite « légère » car possédant une fraction importante en hydrocarbures à courtes chaînes. La seconde (de référence 770350) est une huile « lourde » car possédant une importante fraction d'hydrocarbures à longues chaînes. Enfin, le pétrole « Arabian Light » est un pétrole brut léger provenant d'Arabie Saoudite.

Tout d'abord, des échantillons comprenant des micro-volumes d'huiles d'environ 5μL sont prélevés à l'aide d'une micro-seringue de chromatographie gazeuse et injectés dans la nacelle. Enfin la masse des échantillons est pesée à l'aide d'une balance de précision. De la silice inerte peut-être ajouté si besoin.

La nacelle 3 contenant l'échantillon est alors mise à disposition du passeur automatique qui permet l'introduction de cette dernière dans le four 4 de pyrolyse via le piston 2 (cf figure 1).

L'évolution de la température du four est programmée à l'avance suivant un cycle allant d'une température T1 comprise préférentiellement entre 25°C et 35°C jusqu'à une température finale T2 comprise, préférentiellement mais non exclusivement, entre 640°C et 660°C. La rampe de la variation de température utilisée est comprise préférentiellement entre 15 et 25°C/min (cf figure 2).

Lors de l'augmentation de la température dans le four de pyrolyse, le signal d'hydrocarbures détecté par le FID 6 est enregistré.

Les résultats obtenus pour les huiles et les pétroles sont alors traités via un logiciel spécifique permettant de visualiser et de traiter les résultats d'analyse issus du Rock-Eval. Les données acquises sont alors importées dans le module de distillation simulée Rock-Eval. Selon la position relative de la courbe d'hydrocarbures sur le graphique masse/température (f(T)=m), cette dernière est segmentée en sous-aires ou bien déconvoluée selon une méthode mathématique choisie par l'utilisateur reposant sur les lois expérimentales d'Antoine.

Chacune des aires obtenues corresponds alors à une classe d'hydrocarbures possédant le même nombre d'atomes de carbone et permet alors d'obtenir une estimation de la répartition de ces hydrocarbures selon leur nombre d'atomes de carbone (Cf figure 4).

Enfin, connaissant les températures d'ébullition des n-alcanes correspondant, il est possible de reconstituer les courbes de distillation simulées pour chacune des trois huiles analysées (cf figure 5). La masse molaire moyenne de l'ensemble de l'huile peut également être estimée.

La composition des trois huiles déterminée à l'aide de la méthode de distillation simulée Rock-Eval ainsi que celle obtenue via une analyse par chromatographie gazeuse est représentée par les diagrammes de la figure 6 à 8.

Si l'on compare ces trois huiles, on observe que chacune d'entre elles possède son propre « pattern » en termes de répartition des classes d'hydrocarbures qui résulte directement de sa composition. En effet, l'huile de référence 775199 possède une bimodalité de sa composition avec environ deux tiers d'hydrocarbures légers et un tiers d'hydrocarbures plus lourds ce qui se traduit par un « pattern » spécifique du diagramme de répartition des hydrocarbures ainsi que de sa courbe de distillation simulée. L'huile de référence 770350 est, quant à elle, plus homogène ce qui se traduit par une répartition de type gaussienne des proportions des hydrocarbures la composant. La courbe de distillation simulée correspondante est de type sigmoïdale. Enfin, l'Arabian Light possède une répartition de type gaussienne asymétrique centrée sur l'hydrocarbure en C13 mais avec des proportions d'hydrocarbures lourds variées et allant en diminuant lorsque les chaînes d'hydrocarbures s'allongent.

Afin de pouvoir comparer les résultats de distillation simulée, respectivement, par Rock-Eval et par chromatographie gazeuse, une normalisation est nécessaire sur l'intervalle C1-C40 (limite de caractérisation de la chromatographie utilisée) par rapport aux données du Rock-Eval. En effet, l'analyse Rock-Eval permettant d'augmenter la température jusqu'à 650°C dans le four de pyrolyse, l'intégralité de la quantité d'huile est quantifiée contrairement aux analyses de chromatographies classiques.

Lorsque l'on compare les données de distillation simulée, d'une part, par Rock-Eval et, d'autre part, par chromatographie, on observe que « l'empreinte digitale » de l'huile analysée, qui est matérialisée par son diagramme de répartition des hydrocarbures et sa courbe de distillation simulée, est largement préservée. Quelques différences subsistent néanmoins, toutes relatives aux spécificités des deux méthodes utilisées.

En effet, le dispositif n'étant pas pourvu d'une colonne de séparation, un phénomène de mélange des hydrocarbures analysés se produit. C'est pourquoi les isomères se trouvant loin de l'isomère « moyen » du mélange (c'est-à-dire le plus représenté au sein du mélange) ont des températures d'ébullition affectées et sont déplacées vers les températures moyennes du mélange. Ce phénomène s'observe particulièrement bien sur l'histogramme de répartition Rock-Eval de l'huile de référence 775199 et notamment sur le deuxième pic centré autour de l'hydrocarbure en C21 tandis que ce dernier est centré autour des isomères en C26 sur le diagramme de répartition de la chromatographie.

Une autre limite du procédé de l'invention résulte de la température minimale de démarrage du cycle d'analyse qui correspond à la température ambiante du laboratoire plus 10 °C environ, soit 30°C. Cette température ne permet pas de caractériser les hydrocarbures possédant moins de 7 atomes de carbone. Cependant, la méthode de caractérisation des hydrocarbures en C1-C6 décrite précédemment dans le brevet EP3918321 peut venir compléter la caractérisation des hydrocarbures légers.

## Revendications

1. Procédé pour la caractérisation et la quantification en continu des hydrocarbures en C1-C120 présents dans un échantillon unique prélevé dans un bassin sédimentaire, ledit procédé comprenant la pyrolyse sous atmosphère non oxydante de l'échantillon, l'entraînement des effluents gazeux de pyrolyse au moyen d'un courant de gaz inerte puis l'analyse desdits effluents en vue d'obtenir leurs profils en fonction de la température de pyrolyse, **caractérisé en ce qu'**on effectue la dé-convolution desdits profils en sous aires correspondant à chaque classe d'isomère via un abaque de référence établi via l'équation (F) ci-après pour chacune desdites classes et obtenu en utilisant l'équation empirique d'Antoine issue de l'intégration de la loi physique de Clausius Clapeyron pour une enthalpie de vaporisation supposée constante ;

$$m_i = (D_v \times M_i \times t)/(R \times T) \times 10^{A-B/(T+C)}$$

avec :

$m_i$ (kg) : la masse de l'hydrocarbure considéré
$D_v$ (m$^3$.s$^{-1}$): le débit volumique du gaz vecteur
$M_i$ (kg.mol$^{-1}$) : la masse molaire de l'hydrocarbure considéré
T (K): la température
t (s) : le temps
R (J.mol$^{-1}$.K$^{-1}$): la constante des gaz parfaits
A (Pa), B (-) et C (K) : les paramètres de l'équation d'Antoine
$P_i$ (Pa): la pression partielle

2. Procédé selon la revendication 1, **caractérisé en ce qu'**on effectue la pyrolyse avec une vitesse de chauffe constante comprise entre 0,5°C/mn et 50°C/mn pour atteindre une température finale comprise entre 30°C et 900°C.

3. Procédé selon la revendication 2, **caractérisé en ce qu'**on effectue la pyrolyse selon une vitesse de chauffe comprise entre 15°C/mn et 25°C/min pour atteindre une température finale comprise entre 640°C et 660°C.

4. Procédé selon l'une des revendications précédentes, **caractérisé en ce qu'**on effectue la dé-convolution des profils mesurés par l'équation d'Antoine de façon à obtenir les proportions des classes d'isomères et les courbes de distillation simulée de l'échantillon analysé ainsi que le diagramme de répartition desdites classes d'isomères.

5. Procédé selon l'une des revendications précédentes, **caractérisé en ce que** les effluents gazeux de pyrolyse sont entraînés et dirigés par le courant de gaz inerte avec un débit compris entre 20ml/mn et 70ml/mn.

6. Procédé selon l'une des revendications précédentes, **caractérisé en ce qu'**on effectue l'analyse des effluents au moyen d'un détecteur à ionisation de flamme.

7. Procédé selon l'une des revendications précédentes, **caractérisé en ce que** ledit échantillon est choisi dans le groupe comprenant les roches mères, les kérogènes, les charbons, les roches réservoir, les pétroles bruts et les fractions pétrolières lourdes, les résines, les asphaltènes, les catalyseurs et les prélèvements de sols.

**Patentansprüche**

1. Verfahren für die kontinuierliche Kennzeichnung und Quantifizierung von C1-C120-Kohlenwasserstoffen, die in einer einzelnen Probe vorhanden sind, die aus einem Sedimentbecken entnommen wird, das Verfahren umfassend die Pyrolyse der Probe in einer nicht oxidierenden Atmosphäre, das Mitreißen der gasförmigen Pyrolyseabflüsse mittels eines Inertgasstroms, dann die Analyse der Abflüsse im Hinblick auf das Erhalten ihrer Profile in Abhängigkeit von der Pyrolysetemperatur, **dadurch gekennzeichnet, dass** die Entfaltung der Profile in Unterbereiche, die jeder Isomerenklasse entsprechen, über ein Referenznomogramm durchgeführt wird, das über die nachstehende Gleichung (F) für jede der Klassen erstellt und unter Verwendung der empirischen Antoine-Gleichung erhalten wird, die sich aus der Integration des physikalischen Gesetzes von Clausius Clapeyron für eine konstante angenommene Verdampfungsenthalpie ergibt;

$$m_i = (D_v \times M_i \times t)/(R \times T) \times 10^{A-B/(T+C)}$$

wobei:

$m_i$ (kg): Masse des betrachteten Kohlenwasserstoffs
$D_v$ (m$^3$.s$^{-1}$): Volumendurchfluss des Trägergases
$M_i$ (kg.mol$^{-1}$): Molmasse des betrachteten Kohlenwasserstoffs
T (K): Temperatur
t (s): Zeit
R (J.mol$^{-1}$.K$^{-1}$): Gaskonstante
A (Pa), B (-) und C (K): Parameter der Antoine-Gleichung
$P_i$ (Pa): Partialdruck

2. Verfahren nach Anspruch 1, **dadurch gekennzeichnet, dass** die Pyrolyse mit einer konstanten Heizrate zwischen 0,5 °C/mn und 50 °C/mn zum Erreichen einer Endtemperatur zwischen 30 °C und 900 °C durchgeführt wird.

**3.** Verfahren nach Anspruch 2, **dadurch gekennzeichnet, dass** die Pyrolyse gemäß einer Heizrate zwischen 15 °C/mn und 25 °C/min zum Erreichen einer Endtemperatur zwischen 640 °C und 660 °C durchgeführt wird.

**4.** Verfahren nach einem der vorstehenden Ansprüche, **dadurch gekennzeichnet, dass** die Entfaltung der Profile, die durch die Antoine-Gleichung gemessen werden, durchgeführt wird, um die Anteile der Isomerenklassen und die Kurven einer simulierten Destillation der analysierten Probe sowie das Verteilungsdiagramm der Isomerenklassen zu erhalten.

**5.** Verfahren nach einem der vorstehenden Ansprüche, **dadurch gekennzeichnet, dass** die gasförmigen Pyrolyseabflüsse durch den Inertgasstrom mit einer Durchflussrate zwischen 20 ml/mn und 70 ml/mn mitgerissen und geleitet werden.

**6.** Verfahren nach einem der vorstehenden Ansprüche, **dadurch gekennzeichnet, dass** die Analyse der Abflüsse mittels eines Flammenionisationsdetektors durchgeführt wird.

**7.** Verfahren nach einem der vorstehenden Ansprüche, **dadurch gekennzeichnet, dass** die Probe aus der Gruppe umfassend Muttergesteine, Kerogene, Kohlen, Speichergesteine, Rohöle und Schwerölfraktionen, Harze, Asphaltene, Katalysatoren und Bodenentnahmen ausgewählt wird.

**Claims**

**1.** A method for the continuous characterization and quantification of C1-C120 hydrocarbons present in a single sample taken from a sedimentary basin, said method comprising pyrolysis under a non-oxidizing atmosphere of the sample, the entrainment of the gaseous pyrolysis effluents by means of an inert gas stream and then the analysis of said effluents in order to obtain their profiles as a function of the pyrolysis temperature, **characterized in that** said profiles are deconvolved into sub-areas corresponding to each class of isomer via a reference chart established via the equation (F) below for each of said classes and obtained using the empirical Antoine equation derived from the integration of the Clausius-Clapeyron physical law for an assumed constant enthalpy of vaporization;

$$m_i = (D_v \times M_i \times t)/(R \times T) \times 10^{A-B/(T+C)}$$

with:

$m_i$ (kg): mass of the hydrocarbon considered

$D_v$ (m$^3$.s$^{-1}$): volume flow rate of the carrier gas
$M_i$ (kg.mol$^{-1}$): molar mass of the hydrocarbon considered
T (K): temperature
t (s): time
R (J.mol$^{-1}$.K$^{-1}$): ideal gas constant
A (Pa), B (-) and C (K): parameters of the Antoine equation
$P_i$ (Pa): partial pressure

**2.** The method according to claim 1, **characterized in that** pyrolysis is carried out with a constant heating rate of between 0.5°C/min and 50°C/min in order to achieve a final temperature of between 30°C and 900°C.

**3.** The method according to claim 2, **characterized in that** pyrolysis is carried out according to a heating rate of between 15°C/min and 25°C/min in order to achieve a final temperature of between 640°C and 660°C.

**4.** The method according to one of the preceding claims, **characterized in that** the measured profiles are deconvolved by the Antoine equation in order to obtain the proportions of the isomer classes and the simulated distillation curves of the analyzed sample, as well as the distribution diagram of said isomer classes.

**5.** The method according to one of the preceding claims,
**characterized in that** the gaseous pyrolysis effluents are entrained and directed by the inert gas stream at a flow rate of between 20 ml/min and 70 ml/min.

**6.** The method according to one of the preceding claims, **characterized in that** the effluents are analyzed by means of a flame ionization detector.

**7.** The method according to one of the preceding claims,
**characterized in that** said sample is chosen from the group comprising source rocks, kerogen, coal, reservoir rocks, crude oils and heavy petroleum fractions, resins, asphaltenes, catalysts and soil samples.

[FIG. 1]

[FIG. 2]

[FIG. 3]

[FIG. 4]

[FIG. 5]

[FIG. 6]

[FIG. 7]

[FIG. 8]

[FIG. 9]

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

**Documents brevets cités dans la description**

- FR 2472754
- EP 0691540 B1
- EP 3918321 A
- EP 2342557 A